# EUROPÄISCHE PATENTSCHRIFT

(11) **EP 0 973 713 B1**
(45) Veröffentlichungstag und Bekanntmachung des Hinweises auf die Patenterteilung: **12.06.2002**
(21) Anmeldenummer: 98919174.7
(22) Anmeldetag: 27.03.1998
(51) Int. Cl.: C07C 45/42, C07C 45/45, C07C 47/228, C07C 47/232, A61K 7/46

(54) **VERFAHREN ZUR HERSTELLUNG AROMATISCHER ALDEHYDE**
METHOD FOR PRODUCING AROMATIC ALDEHYDES
PROCEDE DE PREPARATION D'ALDEHYDES AROMATIQUES

(30) Priorität: 05.04.1997 DE 19714042
(43) Veröffentlichungstag der Anmeldung: 26.01.2000
(73) Patentinhaber: Cognis Deutschland GmbH & Co. KG, 40589 Düsseldorf (DE)
(72) Erfinder: MARKERT, Thomas, D-40789 Monheim (DE); NEMITZ, Ralph, D-41363 Jüchen (DE)
(86) Internationale Anmeldenummer: EP9801820
(87) Internationale Veröffentlichungsnummer: WO9845237

(56) Entgegenhaltungen:
- DE-A- 2 340 812
- CHEMICAL ABSTRACTS, vol. 99, no. 21, 21.November 1983 Columbus, Ohio, US; abstract no. 175309d, GULYI ET AL.: "production of cis and trans isomers of beta and n-alkylcinnamaldehydes" Seite 580; XP002071096 & MASLO-ZHIR.PROM.ST., Bd. 7, 1983, Seiten 32-34,
- SCHMIDLE ET AL.: "The preparation of cinnamaldehydes by the formylation of styrenes " JOURNAL OF AMERICAN CHEMICAL SOCIETY, Bd. 78, 1956, Seiten 3209-10, XP002071394
- "Riechstoffe und Geruchssinn" von G. Ohloff, Berlin 1990, S. 17
- "Common Fragance and Flavour Materials", K.Bauer et al., VCH 1990, S. 3,4
- Dragoco Report 1983 (83), S. 139-142,144-146,148-150, "Holzriechstoffe" von E.-J- Brunke

## Beschreibung

### Gebiet der Erfindung

Die Erfindung betrifft ein Verfahren zur Herstellung aromatischer Aldehyde aus Phenonketalen spezieller Struktur. Dabei wird zunächst das Phenonketal in Gegenwart von Lewissäuren mit einem Vinylether kondensiert, das entstehende Acetal einer sauren Hydrolyse zum entsprechenden α,β-ungesättigten Aldehyd unterworfen und die C=C-Doppelbindung dieses Aldehyds gewünschtenfalls auf übliche Weise selektiv hydriert.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5.000 kg Rosenblüten notwendig; die Folgen sind eine sehr stark limitierte Weltjahresproduktion sowie ein hoher Preis. Es ist daher klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf an Geruchsverbesserern für Produkte des täglichen Bedarfs wie Kosmetika und Reinigungsmittel decken zu können.

Es ist daher klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf an Geruchsverbesserern für Produkte des täglichen Bedarfs wie Kosmetika und Reinigungsmittel decken zu können.

Darüber hinaus besteht generell ein ständiger Bedarf an synthetischen Riechstoffen, die sich günstig und mit gleichbleibender Qualität herstellen lassen und erwünschte olfaktorische Eigenschaften haben, d.h. angenehme, möglichst naturnahe und qualitativ neuartige Geruchsprofile von ausreichender Intensität besitzen und in der Lage sind, den Duft von kosmetischen und Verbrauchsgütern vorteilhaft zu beeinflussen.

In diesem Zusammenhang werden an die Reinheit und damit im Zusammenhang stehend an das Geruchsprofil synthetischer Riechstoffe zunehmend höhere Anforderungen gestellt. Dies bedeutet; daß die Entwicklung von Verfahren, durch die synthetische Riechstoffe mit verbesserter Reinheit und verbesserter Geruchscharakteristik zunehmend an Bedeutung gewinnt. Dabei haben solche verbesserten Herstellverfahren in der Regel den zusätzlichen Vorteil, daß die so hergestellten Riechstoffe aufgrund ihrer höheren Reinheit auch günstigere ökotoxikologische Eigenschaften aufweisen.

Ein besonderer Bedarf besteht in der Fachwelt derzeit nach Riechstoffen, die über eine ausgeprägte Moschus-Geruchscharakteristik verfügen.

4-tert.-Butylvinylpropionaldehyd sowie sein α-Methyl-Analogon sind US **626548** als mögliche Riechstoff-Komponenten bekannt. Sie sind gemäß US 626548 zugänglich, indem α-Methyl-Styrol in Gegenwart von Rhodium- oder Cobalt-Katalysatoren mit Wasserstoff und Kohlenmonoxyd im Sinne einer Hydroformulierungsreaktion umgesetzt werden.

DE-A-23 40 812 offenbart spezielle phenylpropanale sowie ein Verfahren zu deren Herstellung.

### Beschreibung der Erfindung

Aufgabe der vorliegenden Erfindung war es, ein verbessertes Verfahren zur Herstellung aromatischer Aldehyde mit Riechstoffeigenschaften bereitzustellen.

Gegenstand der Erfindung ist ein Verfahren zur Herstellung aromatischer Aldehyde mit Riechstoffeigenschaften der allgemeinen Struktur (I) 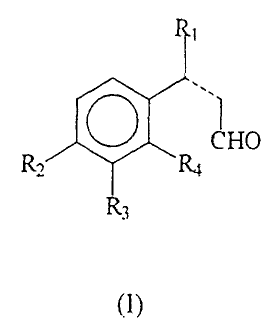 worin R₁ eine Methyl-, Ethyl- oder Propylgruppe, R₂ eine Methyl-, Ethyl-. Isopropyl-, tert.-Butyl- oder Methoxygruppe, R₃ Wasserstoff oder eine Methoxygruppe und R₄ Wasserstoff oder eine Methylgruppe bedeuten und worin die gestrichelte Linie eine C-C-Einfachbindung oder eine C=C-Doppelbindung, die E- oder Z-konfiguriert sein kann, bedeutet,
wobei man
a) zunächst ein Phenonketal der allgemeinen Struktur (II) 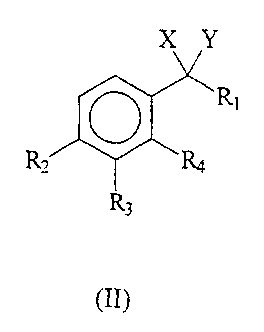 worin X und Y unabhängig voneinander Methoxy-, Ethoxy-, Propoxy- oder Butoxyreste und R₁ eine Methyl-, Ethyl- oder Propylgruppe, R₂ eine Methyl-, Ethyl-, Isopropyl-, tert.-Butyl- oder Methoxygruppe, R₃ Wasserstoff oder eine Methoxygruppe und R₄ Wasserstoff oder eine Methylgruppe bedeuten, in Gegenwart von Lewissäuren an einen Vinylether der allgemeinen Struktur (III) 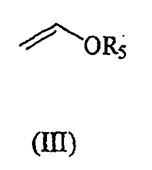 worin R₅ einen Alkylrest mit 1 bis 4 C-Atomen bedeutet, addiert,
b) das entstehende Acetal einer sauren Hydrolyse zum entsprechenden α,β-ungesättigten Aldehyd unterwirft und
c) die C=C-Doppelbindung dieses Aldehyds gewünschtenfalls auf übliche Weise selektiv hydriert.

In der Stufe a) ist es bevorzugt, als Lewissäure-Katalysator Zinkchlorid einzusetzen.

Nach dem erfindungsgemäßen Verfahren können die Verbindungen (I) entweder in Reinsubstanz oder im Gemisch untereinander hergestellt werden.

Die nach dem erfindungsgemäßen Verfahren hergestellten aromatischen Aldehyde (I) zeichnen sich gegenüber entsprechenden Verbindungen, die durch Hydroformulierung entsprechender Styrole gemäß dem bekannten Stand der Technik hergestellt wurden, durch eine verbesserte Reinheit und eine verbesserte Geruchscharakteristik aus.

In einer weiteren Ausführungsform betrifft die Erfindung die Verwendung von 3-(2,4-Dimethylphenyl)-butanal als Riechstoff.

In einer bevorzugten Ausführungsform der vorliegenden Erfindung ist der Hydrierungsschritt c) obligatorisch.

Die Einzelschritte der Reaktionssequenz a) bis c) des erfindungsgemäßen Verfahrens sind an sich literaturbekannt.

So ist beispielsweise die Addition von Ketalen an Vinylether in Gegenwart von Bortrifluorid-etherat aus **Houben-Weyl**. Methoden der organischen Chemie, 4. Auflage, Band VII, Teil 1, Seiten 112 und 115-117 (Stuttgart 1954) bekannt.

In **J. Org. Chem**., Vol.43, No. 10, 1978, Seiten 2068-2069 wird im Zusammenhang mit einer Syntheseroute zur Herstellung von Pheromonen 1,1-Dioxyethyl-3,3-dimethyl-cyclohexan mit Ethylvinylether in Gegenwart von Zinkchlorid in das entsprechende Acetal überführt und dieses Acetal mit Essigsäure/Natriumacetat/Wasser zu den entsprechenden isomeren ungesättigten Aldehyden hydrolysiert.

Ein weiterer Gegenstand der Erfindung ist die neue Substanz 3-(2,4-Dimethylphenyl)-butanal.

Die folgenden Beispiele dienen der Erläuterung der Erfindung und sind nicht einschränkend zu verstehen.

### Beispiele

### Vorprodukt 1: -(1,1-Diethoxyethyl)-2,4-dimethylbenzol

### Ansatz:

1) 97.3 g (0.66 Mol) 2,4-Dimethylacetophenon (Fa. Fluka)
2) 117,3 g (0,79 Mol) Triethylorthoformiat (Fa. Fluka)
3) 123,7 mg Kaliumhydrogensulfat (Fa. Merck)
4) 600 ml Ethanol, wasserfrei (technische Ware)

### Ausführung:

Unter Feuchtigkeitsausschluß (mit Kieselgel gefülltes Trockenrohr) wurden die Substanzen 1) bis 4) bei 20 °C unter Durchleiten von ca 2 1/h trockenem Stickstoff in einem 1-Dreihalskolben über 78 Stunden gerührt. Danach war über GLC kein Edukt mehr im Gemisch nachweisbar. Der Katalysator wurde zur Aufarbeitung mit 2 ml Natriummethylatlösung (30 % in Methanol) neutralisiert und die Mischung 15 Minuten gerührt. Danach wurde das Ethanol am Rotationsverdampfer abgezogen und die verbleibenden 150,9 g hellbrauner Rückstand zur Destillation an einer 15 cm Füllkörper-Kolonne (Braunschweiger Wendeln) eingesetzt. Es wurden 133,5 g Hauptlauf mit einem Siedepunkt von 73 - 75 °C/0,1 mbar erhalten. Die gaschromatographisch bestimmte Reinheit betrug 94,6 %.

### Geruchsbeschreibung: Moschus-, Anthranilat-, Schwefelkautschuk-Note

### Vorprodukt 2: 2,4-Dimethyl-1-(1,3,3-triethoxy-1-methyl-propyl)-benzol

### Ansatz:

1) 127,6 g (0,57 Mol) 1-(1,1-Diethoxyethyl)-2,4-dimethyl-benzol, hergestellt wie oben unter "Vorprodukt 1" beschrieben
2) 44,9 ml ZnCl ₂-Lösung (10 % in Essigsäureethylester)
3) 59,5 ml (0,65 Mol) Ethylvinylether (Fa. Acros)

### Ausführung:

Das Ketal 1) und die Zink(II)chlorid-Lösung wurden in einem 1-1-Dreihalskolben unter Rühren und Spülen mit trockenem Stickstoff auf 40 - 45 °C erwärmt. Der Ethylvinylether wird bei ca. 50 ° C innerhalb einer Stunde kontinuierlich zugetropft und ca. 15 Stunden bei 50 °C weiter gerührt. Danach war das Ausgangsketal vollständig umgesetzt. Zur Aufarbeitung wurde mit Diethylether verdünnt und mit ja ca. 150 ml 5%iger Natronlauge und Wasser gewaschen, die organischen Phasen über Natriumsulfat getrocknet und eingeengt. Die verbleibenden 146,7 g gelbe Flüssigkeit wurden zur Destillation an einer 15 cm Füllkörper-Kolonne eingesetzt. 90,4 g Hauptlauf mit einem Siedepunkt von 101 - 105 °C und einer gaschromatographisch bestimmten Reinheit von 96,6 % wurden bei dieser Reinigungsoperation erhalten.

**Geruchsbeschreibung:** blumig, süß, Zimt-Note

### Beispiel 1: 3-(2,4-Dimethylphenyl)-but-2-enal

### Ansatz:

1) 70.5 g (0.25 Mol) 2,4-Dimethyl-l-(1,3,3-triethoxy-1-methyl propyl)-benzol, hergestellt wie oben unter "Vorprodukt 2" beschrieben
2) 342.9 g (5,69 Mol) Essigsäure, 99%ig (Fa. Riedel de Haen)
3) 35,7 g (0,44 Mol) Natriumacetat (Fa. Merck)
4) 24,1 g dest Wasser

### Ausführung:

In einem 1l Dreihalskolben mit Rührer, Thermometer und Rückflußkühler wurden die Komponenten l) bis 4) vorgelegt und 3 Stunden bei 90 - 95 °C gerührt. Anschließend wurde das Heizbad entfernt und unter Rühren über Nacht auf Raumtemperatur kommen gelassen. Es wurde ca. 200 ml Diethylether zugesetzt und das Gemisch auf ca. 500 g Eis gegossen. Unter Rühren wurde nun die stark schäumende Mischung im 2 l Becherglas mit festem Natriumbicarbonat neutralisiert. Die organische Phase wurde abgetrennt, mit ca. 150 ml Wasser nachgewaschen, über Natriumsulfat getrocknet und eingeengt. 45,3 % eines dunkelbraunen Rückstands wurden zur Destillation an einer 30 cm langen Füllkörperkolonne eingesetzt. Der Hauptlauf bestand aus 29,4 g leicht gelblicher Flüssigkeit mit einem Siedepunkt von 76 °C/0,08 mbar und einer gaschromatographisch bestimmten Reinheit von 95.3 %.

**Geruchsbeschreibung:** blumig, Cedemholz, stechend aldehydisch.

### Beispiel 2: 3-(2,4-Dimethylphenyl)-butanal

### Ansatz:

1) 19.5 g (0,11 Mol) 3-(2,4-Dimethylphenyl)-but-2-enal, hergestellt gemäß Beispiel 1
2) 0.2 g 5 % Pd/C (RCh-Katalysator 50/8, Fa. Hoechst)
3) 200 ml Ethanol (technische Ware)

### Ausführung:

Die Komponenten wurden zusammen in einem Stahlautoklaven vorgelegt und 5 Stunden bei 70 °C unter 20 bar Wasserstoff gerührt. Ein nennenswerter Druckabfall wurde nicht beobachtet. Nach Abfiltrieren des Katalysators und Einengen der ethanolischen Lösung wurden 18,5 g farblose Flüssigkeit zur Destillation an einer 30 cm Füllkörperkolonne eingesetzt.
Mit einem Siedepunkt von 62 - 63 °C gingen 10,3 g Hauptlauf über. Die gaschromatographisch bestimmte Reinheit betrug 93 %.

**Geruchsbeschreibung**: blumig, Liguster-, Anthranilat, Nitromoschus-Note.

## Patentansprüche

1. Verfahren zur Herstellung aromatischer Aldehyde mit Riechstoffeigenschaften der allgemeinen Struktur (I) 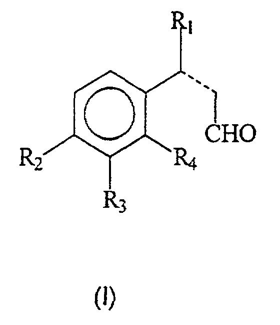 worin R₁ eine Methyl-, Ethyl- oder Propylgruppe, R₂ eine Methyl-, Ethyl-, Isopropyl-, tert.-Butyl- oder Methoxygruppe, R₃ Wasserstoff oder eine Methoxygruppe und R₄ Wasserstoff oder eine Methylgruppe bedeuten und worin die gestrichelte Linie eine C-C-Einfachbindung oder eine C=C-Doppelbindung, die E- oder Z-konfiguiert sein kann, bedeutet,
**dadurch gekennzeichnet, daß** man
a) zunächst ein Phenonketal der allgemeinen Struktur (II) 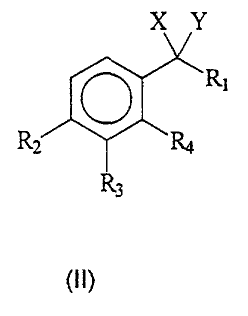 worin X und Y unabhängig voneinander Methoxy-, Ethoxy-, Propoxy- oder Butoxyreste und R₁ eine Methyl-, Ethyl- oder Propylgruppe, R₂ eine Methyl-, Ethyl-, Isopropyl-, tert.-Butyl- oder Methoxygruppe, R₃ Wasserstoff oder eine Methoxygruppe und R₄ Wasserstoff oder eine Methylgruppe bedeuten, in Gegenwart von Lewissäuren an einen Vinylether der allgemeinen Struktur (III) 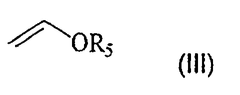 worin R₅ einen Alkylrest mit 1 bis 4 C-Atomen bedeutet, addiert,
b) das entstehende Acetal einer sauren Hydrolyse zum entsprechenden α,β-ungesättigten Aldehyd unterwirft und
c) die C=C-Doppelbindung dieses Aldehyds gewünschtenfalls auf übliche Weise selektiv hydriert.

2. Verfahren nach Anspruch 1, wobei man in der Stufe a) als Lewissäure-Katalysator Zinkchlorid einsetzt.

3. Verfahren nach Anspruch 1 oder 2, wobei man Phenonketale (II) einsetzt, bei denen die Reste X und Y jeweils Ethoxygruppen sind.

4. Verfahren nach einem der Ansprüche 1 bis 3, wobei man ais Vinylether (III) Ethylvinylether einsetzt.

5. Verfahren nach einem der Ansprüche 1 bis 4, wobei man in Stufe b) die saure Hydrolyse in Gegenwart von Essigsäure durchführt.

6. Verwendung von 3-(2,4-Dimethylphenyl)-butanal als Riechstoff.

7. 3-(2,4-Dimethylphenyl)-butanal.

## Claims

1. A process for the production of aromatic aldehydes having perfume properties corresponding to general formula (I): 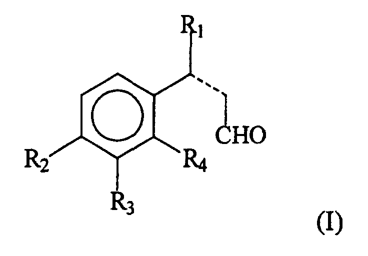 where R₁ is a methyl, ethyl or propyl group, R₂ is a methyl, ethyl, isopropyl, tert.butyl or methoxy group, R₃ is hydrogen or a methoxy group and R₄ is hydrogen or a methyl group and where the chain line represents a C-C single bond or C=C double bond which may have the E or Z configuration,
**characterized in that**
a) a phenone ketal corresponding to general formula (II): 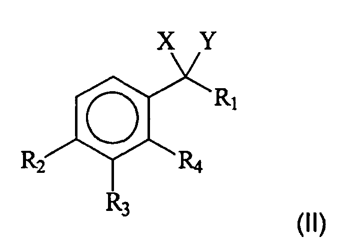 where X and Y independently of one another represent methoxy, ethoxy, propoxy or butoxy groups and R₁ is a methyl, ethyl or propyl group, R₂ is a methyl, ethyl, isopropyl, tert.butyl or methoxy group, R₃ is hydrogen or a methoxy group and R₄ is hydrogen or a methyl group, are added onto a vinyl ether corresponding to general formula (III): 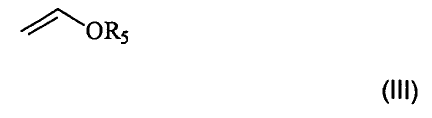 where R₅ is an alkyl group containing 1 to 4 carbon atoms, in the presence of Lewis acids,
b) the acetal formed is subjected to acidic hydrolysis to form the corresponding α,β-unsaturated aldehyde and
c) if desired, the C=C double bond of this aldehyde is selectively hydrogenated in the usual way.

2. A process as claimed in claim 1, **characterized in that** zinc chloride is used as the Lewis acid catalyst in step a).

3. A process as claimed in claim 1 or 2, **characterized in that** phenone ketals (II) in which X and Y are both ethoxy groups are used.

4. A process as claimed in any of claims 1 to 3, **characterized in that** ethylvinyl ether is used as the vinyl ether (III).

5. A process as claimed in any of claims 1 to 4, **characterized in that** the acidic hydrolysis step b) is carried out in the presence of acetic acid.

6. The use of 3-(2,4-dimethylphenyl)-butanal as a perfume.

7. 3-(2,4-dimethylphenyl)-butanal.

## Revendications

1. Procédé pour la préparation d'aldéhydes aromatiques à propriétés de parfums, de formule générale (I) 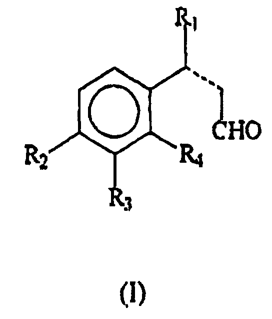 dans laquelle R₁ représente un groupe méthyle, éthyle ou propyle. R₂ représente un groupe méthyle, éthyle, isopropyle, tent-butyle ou méthoxy, R₃ représente un atome d'hydrogène ou un groupe méthoxy, et R₄ représente un atome d'hydrogène ou un groupe méthyle, et dans laquelle le trait interrompu représente une liaison simple C-C ou une double liaison C=C, qui peut avoir la configuration E ou Z,
**caractérisé en ce que**
a) on fixe d'abord par addition un phénone-cétal de formule générale (II) 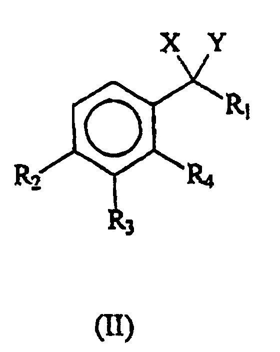 dans laquelle X et Y représentent, indépendamment l'un de l'autre, des radicaux méthoxy, éthoxy, propoxy ou butoxy et R₁ représente un groupe méthyle, éthyle ou propyle, R₂ représente un groupe méthyle, éthyle, isopropyle, tert-butyle ou méthoxy, R₃ représente un atome d'hydrogène ou un groupe méthoxy. et R₄ représente un atome d'hydrogène ou un groupe méthyle, en présence d'acides de Lewis, sur un éther vinylique de formule générale (III) 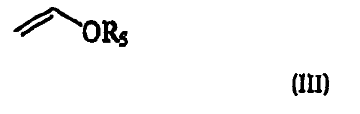 dans laquelle R₅ représente un radical alkyle ayant de 1 à 4 atomes de carbone,
b) on soumet l'acétal formé à une hydrolyse acide conduisant à l'aidéhyde α,β-insaturé correspondant, et
c) on soumet si on le désire la double liaison C=C de cet aldéhyde à une hydrogénation sélective, à la manière usuelle.

2. Procédé selon la revendication 1,
dans lequel comme catalyseur de type acide de Lewis dans l'étape a), on utilise du chlorure de zinc.

3. Procédé selon la revendication 1 ou 2.
dans lequel on utilise des phénone-cétals (II) dans lesquels les radicaux X et Y représentent chacun un groupe éthoxy.

4. Procédé selon l'une quelconque des revendications 1 à 3,
dans lequel on utilise en tant qu'éther vinylique (III) de l'oxyde d'éthyle et de vinyle.

5. Procédé selon l'une quelconque des revendications 1 à 4,
dans lequel on effectue dans l'étape b) l'hydrolyse acide en présence d'acide acétique.

6. Utilisation du 3-(2,4-diméthylphényl)butanal en tant que parfum.

7. 3-(2,4-diméthylphényl)butanal.
